# FASCICULE DE BREVET EUROPEEN

(11) **EP 3 242 674 B1**
(45) Date de publication et mention de la délivrance du brevet: **10.11.2021**
(21) Numéro de dépôt: 15732710.7
(22) Date de dépôt: 30.06.2015
(51) Int. Cl.: A61K 36/9066, A61K 35/748, A61P 9/00, A61P 25/00, A61P 3/10

(54) **COMPOSITIONS CONTENANT DE LA CURCUMINE À BIODISPONIBILITÉ AMÉLIORÉE**
ZUSAMMENSETZUNGEN MIT KURKUMA MIT VERBESSERTER BIOVERFÜGBARKEIT
COMPOSITIONS CONTAINING CURCUMIN HAVING IMPROVED BIOAVAILABILITY

(30) Priorité: 07.01.2015 LU 92630
(43) Date de publication de la demande: 15.11.2017
(73) Titulaire: Fresu, Gianluca, 27670 Le Bosc Roger en Roumois (FR)
(72) Inventeur: FRESU, Antonello, L-6914 Roodt Sur Syre (LU)
(74) Mandataire: Office Freylinger
(86) Numéro de dépôt international: PCT/EP2015/064855
(87) Numéro de publication internationale: WO 2016/110334

(56) Documents cités:
- DE-A1- 19 906 016
- FR-A1- 2 949 646
- US-A1- 2007 122 496
- US-A1- 2008 260 695
- US-A1- 2012 251 500
- None

## Description

### Domaine technique

La présente invention concerne d'une manière générale des compositions comprenant de la curcumine, dont la biodisponibilité est nettement améliorée par rapport aux compositions connues.

### Etat de la technique

La curcumine fait partie des phénols naturels et est utilisée depuis longtemps non seulement dans le domaine alimentaire (principalement comme colorant), mais également pour ses effets bénéfiques sur la santé. En effet, la curcumine est un antioxydant très actif, un antiseptique et antibactérien efficace, ainsi qu'un puissant anti-inflammatoire. D'autres effets bénéfiques sont cités dans la littérature, notamment sur diverses maladies humaines et animales, notamment le cancer, les maladies cardiovasculaires, le diabète, l'arthrite, les maladies neurologiques et la maladie de Crohn, entre autres.

Malheureusement, il a été constaté que lorsqu'elle est utilisée seule, la curcumine est très rapidement éliminée par l'organisme. Différents travaux effectués chez l'homme et le rat notamment ont mis en évidence que la curcumine administrée par voie orale présente une biodisponibilité limitée tant à cause de la faible absorption intestinale, qu'à cause de l'excrétion rénale et biliaire élevée. D'autres études ont mis en évidence que l'administration par voie intraveineuse de curcumine chez le rat est suivie d'une excrétion de la même de plus de 50% par voie biliaire en 5 heures.

Dans le domaine de la pharmacie (mais également en nutrition), le terme « biodisponibilité » est utilisé pour décrire une propriété pharmacocinétique d'un composé, à savoir la fraction d'une dose qui atteint la circulation sanguine sous forme inchangée. C'est un concept qui est donc essentiel en pharmacocinétique, car la biodisponibilité doit être prise en considération lors du calcul des doses pour des voies d'administration autres qu'intraveineuse.

Concernant la curcumine, il est également connu qu'elle présente un effet laxatif au-delà de certaines concentrations sanguines chez beaucoup d'espèces animales, notamment aussi chez l'homme. Il est donc important que toute solution proposée permette d'améliorer la biodisponibilité, mais sans augmenter les taux de curcumine au-delà des concentrations gênantes.

De nombreuses tentatives ont en effet eu comme objectif de remédier au problème de la biodisponibilité réduite de la curcumine. Citons par exemple, l'utilisation de la pipérine (présente dans le poivre noir) qui serait capable d'inhiber les voies d'élimination de la curcumine, et ainsi de multiplier sa biodisponibilité par un facteur 20 (Shoba G, Joy D, Joseph T, Majeed M, Rajendran R, Srinivas PS. Influence of piperine on the pharmacokinetics of curcumin in animais and human volunteers. Planta Med. 1998 May;64(4):353-6.). Cependant, même si la biodisponibilité est améliorée, les valeurs de taux sanguins ne sont pas optimales et la pipérine présente un effet irritant sur l'intestin.

D'autres pistes suivies pour contrer la faible biodisponibilité comprennent notamment l'utilisation de la curcumine liposomale; la préparation de nanoparticules de curcumine; l'utilisation de complexes curcumine-phospholipide; et l'utilisation d'analogues structuraux de la curcumine.

### Objet de l'invention

Un objet de la présente invention est par conséquent de proposer des compositions comprenant de la curcumine, qui permettent d'améliorer la biodisponibilité de cette dernière par comparaison à sa biodisponibilité de la curcumine prise seule, ces compositions pouvant servir le cas échéant d'alternative ou de complément aux solutions existantes.

### Description générale de l'invention

Afin de résoudre le problème mentionné ci-dessus, la présente invention propose, dans un premier aspect, l'utilisation non-thérapeutique d'une quantité efficace de spiruline et/ou d'extrait(s) de spiruline pour améliorer la biodisponibilité de la curcumine administrée ou prise par voie orale chez l'animal, en particulier les animaux domestiques ou de compagnie, tels que le cheval, le porc, le chien, le chat ; ou chez l'homme et dans laquelle la curcumine est utilisée à titre de complément alimentaire dans le domaine de l'alimentation animale ou humaine.

L'invention propose également une préparation thérapeutique orale à biodisponibilité améliorée de la curcumine consistant en une combinaison de curcumine avec une quantité efficace de spiruline et/ou un ou des extrait(s) de spiruline.

Dans un aspect supplémentaire, l'invention concerne aussi des préparations orales à biodisponibilité améliorée de la curcumine comprenant de la curcumine et une quantité efficace de spiruline ou d'extrait(s) de spiruline, éventuellement en combinaison avec d'autres ingrédients actifs, excipients ou supports, pour l'utilisation pour le traitement ou la prévention de maladies humaines et animales, choisies parmi les maladies cardiovasculaires, le diabète, les maladies neurologiques et la maladie de Crohn.

Dans un aspect supplémentaire, l'invention concerne une préparation non-thérapeutique orale à biodisponibilité améliorée de la curcumine, notamment un complément alimentaire, consistant en une combinaison de curcumine avec une quantité efficace de spiruline et/ou un ou des extrait(s) de spiruline.

En effet, de manière surprenante pour l'homme de métier, les inventeurs ont constaté que le fait de combiner la curcumine à la spiruline permet d'augmenter nettement la biodisponibilité de la curcumine lorsque celle-ci est prise par voie orale. De plus, il a été montré que l'invention permet non seulement d'augmenter cette biodisponibilité, mais également de contrôler la concentration maximale dans le sang et de la maintenir à un niveau suffisant pendant une période plus longue, c'est-à-dire d'augmenter la fraction de la curcumine qui est absorbé au niveau de l'intestin, mais en même temps d'allonger le temps de présence de la curcumine à des concentrations utiles.

S'il est vrai que la spiruline et les extraits de spiruline sont connus depuis longtemps comme aliment ou complément alimentaire à cause de leur valeur nutritionnelle élevée, son effet bénéfique sur la biodisponibilité de la curcumine prise par voie orale est resté inconnu jusqu'ici.

En fait, d'une manière générale, le terme «spiruline» est utilisé pour désigner un certain nombre de cyanobactéries différentes du genre *Arthrospira,* notamment « *Arthrospira platensis* » (parfois désigné *Spirulina platensis*), mais aussi *Arthrospira maxima, Arthrospira pacifica,* etc. Ce terme désigne également des préparations à base de ces cyanobactéries, en l'occurrence comme aliment, mais la plupart du temps comme compléments alimentaires, par exemple sous forme séchée et comprimée. Dans le contexte de la présente invention, le terme « spiruline » peut désigner indifféremment les cyanobactéries ou des préparations les contenant selon le contexte, étant entendu que les indications quantitatives relatives à la spiruline se rapportent au poids sec effectif de cyanobactéries du genre *Arthrospira.* Les termes « extrait de spiruline » ou « extraits de spiruline » dans le contexte de la présente invention désignent un ou plusieurs extraits obtenus à partir de spiruline telle que définie ci-dessus, c'est-à-dire un ou plusieurs extraits de cyanobactéries du genre *Arthrospira*, l'extrait ayant été obtenu par un procédé d'extraction connu, de préférence il s'agit d'un extrait à l'éthanol. Les indications quantitatives concernant les extraits de spiruline se réfèrent, sauf indication explicite contraire, au poids sec de l'extrait.

Le terme « quantité efficace » dans le contexte de la présente invention signifie une quantité de spiruline et/ou d'extrait(s) de spiruline qui permet d'avoir un effet positif notable et significatif sur la biodisponibilité de la curcumine prise par voie orale. Bien que la valeur réelle de cette quantité efficace puisse varier notamment en fonction de l'animal considéré ou des autres ingrédients éventuels de la préparation, il est aisé pour l'homme de métier de vérifier la quantité effectivement nécessaire pour obtenir l'effet désiré dans une situation donnée. De préférence, cette quantité efficace est exprimée comme rapport pondéral de spiruline ou d'extrait de spiruline à la quantité de curcumine dont elle améliore la biodisponibilité. De préférence, ce rapport pondéral spiruline : curcumine (respectivement extrait(s) de spiruline : curcumine) est compris entre 20 : 1 et 1 : 3, de manière davantage préférée entre 10 : 2 et 1 : 2, en particulier entre 8 : 3 et 3 : 4.

La quantité de curcumine contenue dans les préparations selon l'invention est bien entendu fonction de nombreux facteurs, dont l'effet recherché, respectivement l'indication à traiter, l'animal considéré, sa taille/son poids, la présence d'autres ingrédients ou principes actifs, etc.

Dans la pratique, les quantités de curcumine à utiliser se situent souvent entre 0,1 et 20 mg/kg, de préférence entre 0,5 et 15 mg/kg, de manière davantage préférée entre 1 et 10 mg/kg de poids corporel, de manière tout à fait préférée entre 3,5 et 7,5 mg/kg.

Il est clair qu'en fonction de l'utilité de la préparation et de l'animal à traiter la préparation contiendra d'autres ingrédients actifs ou non et la concentration de curcumine dans la préparation peut donc largement varier.

La présente invention envisage des préparations orales prêtes à l'emploi, mais également des préparations concentrées à mélanger aux aliments usuels, des préparations liquides ou solides, en poudre, sous forme comprimés, de gélules ou autres. Il est à noter que l'invention prévoit explicitement que la spiruline ou ses extraits ne sont pas obligatoirement pris en même temps ou en mélange avec la curcumine pour avoir l'effet recherché. Il est également possible ou envisageable d'administrer successivement la spiruline et la curcumine (dans l'ordre souhaité, de préférence toutefois d'abord la spiruline), en veillant cependant que les deux administrations se font de préférence endéans 1 heure, en particulier endéans une demi-heure.

Les indications à traiter ou les raisons d'administrer de la curcumine sont nombreuses et les exemples suivants ne sont pas destinés à limiter l'étendue de l'invention, mais plutôt pour l'illustrer.

La présente divulgation concerne des préparations thérapeutiques et non-thérapeutiques comprenant en outre d'autres ingrédients pharmaceutiquement actifs ou avantageux pour la santé. Ces ingrédients peuvent être choisis parmi les agents anti-inflammatoires, les analgésiques, les agents anti-cancer, des vitamines, des acides gras essentiels, etc. De préférence ces ingrédients sont d'origine naturelle, notamment des extraits de saule blanc (*Salix alba*), de boswellia (*Boswellia serrata*),*de* yucca (*Yucca schidigera*) et d'harpagophytum.

Les préparations de la présente divulgation peuvent comprendre également d'autres ingrédients servant notamment à améliorer la composition en termes de formulation, de goût, de consistance, de présentation, notamment de couleur, etc. Ces ingrédients peuvent être choisis parmi ceux généralement connus dans le domaine en fonction du type de préparation souhaitée et de l'objectif prévu.

Il est à noter que les préparations selon la divulgation peuvent également comprendre d'autres composants susceptibles d'améliorer la biodisponibilité de la curcumine, par exemple la pipérine. Il est également envisagé que la curcumine (ou une partie de celle-ci) se trouve sous forme de curcumine liposomale; de nanoparticules de curcumine; de complexes curcumine-phospholipide; et/ou d'analogues structuraux de la curcumine.

La présente invention concerne aussi des préparations thérapeutiques et non-thérapeutiques qui ne comprennent comme ingrédients actifs que de la curcumine et de la spiruline, éventuellement avec des excipients ou supports (acceptables en pharmacie ou en alimentation). De tels préparations consistent de préférence en une combinaison de curcumine avec de la spiruline et/ou un ou des extrait(s) de spiruline. En d'autres mots, l'invention envisage également des compositions comprenant uniquement de la curcumine et une quantité efficace de spiruline (ou de ses extraits).

Finalement, un aspect supplémentaire de la divulgation concerne un procédé de fabrication d'une préparation telle que décrite dans ce document, le procédé comprenant une étape de mélange d'une quantité efficace de spiruline et/ou d'un ou de plusieurs extraits de spiruline avec de la curcumine, éventuellement avec d'autres ingrédients actifs, excipients ou supports acceptables dans le domaine de la pharmacie ou de l'alimentation.

### Brève description des dessins

D'autres particularités et caractéristiques de l'invention ressortiront de la description détaillée de quelques modes de réalisation avantageux présentés ci-dessous, à titre d'illustration, en se référant aux figures annexées. Celles-ci montrent:

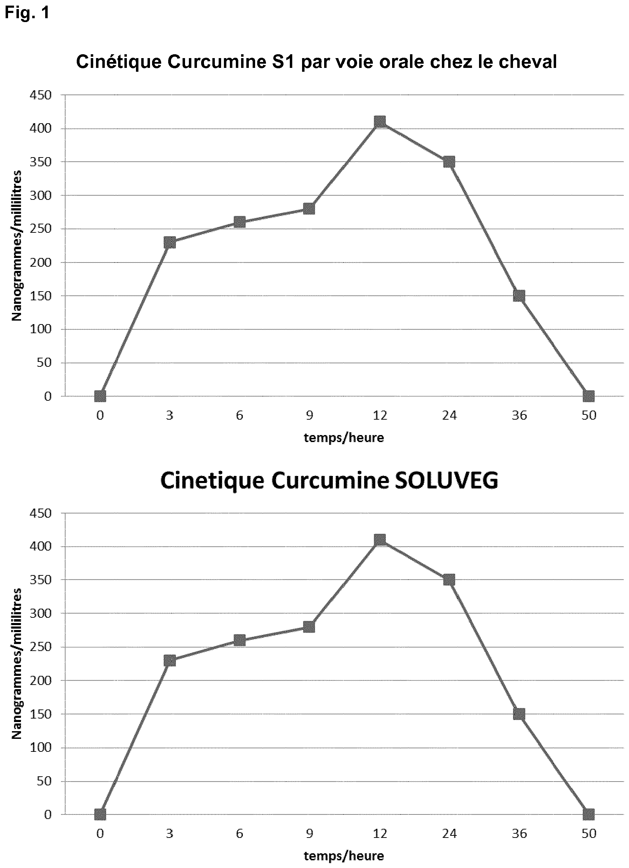

Fig. 1: est un graphe représentant la biodisponibilité (ou la cinétique) de la préparation Curcumine S1 selon l'invention administrée par voie orale chez le cheval;
Fig. 2: est un graphe représentant la biodisponibilité (ou la cinétique) d'une préparation de curcumine administrée par voie intraveineuse chez le porc à titre de comparaison.

### Exemples

### Matériel et méthode

Une première composition comprenant de la curcumine et de la spiruline a été préparée en combinant dans l'ordre sous agitation et homogénéisation constante de l'eau purifiée, du sorbate de potassium, de l'acide citrique, du saccharose, de la curcumine 95% (teneur en curcuminoïdes : 95.7% déterminé par HPLC), de l'extrait de spiruline et de la gomme de xanthane. L'extrait de spiruline a été obtenu en dissolvant 1 g de matière première standard avec 20 ml d'eau à reflux pendant 60 min. Cet extrait à l'eau est filtré, puis concentré à sec dans un dessiccateur sous vide. Le résidu sec est ensuite dissout avec 5 ml de méthanol de manière à obtenir l'extrait de spiruline. Cette préparation est désignée par la suite Curcumine S1.

Un premier essai de détermination de la biodisponibilité (étude pharmacocinétique) de la curcumine chez le cheval a été réalisé en déterminant la quantité de curcumine dans le sang (concentration plasmatique).

Ont été recrutés 3 chevaux entre 7 et 10 ans, avec un poids estimé d'environ 500 kg. La Curcumine S1 est administrée par voir orale (2.6 Grammes de Curcumine S1 pour 500 kg de poids vif, soit 5.6 mg/kg) en une seule prise.

Les échantillons de plasma ont été prélevés avant administration (temps T0) de la préparation Curcumine S1 et 3 heures, 6 heures, 9 heures, 12 heures, 24 heures, 36 heures et 50 heures après l'administration de la préparation.

Les échantillons ont été congelés à la température de -18 C° jusqu'à la détermination analytique.

La technique utilisée pour la détermination de la curcumine plasmatique est la HPLC (Chromatographie Liquide Haute Performance).

### Résultats

Les résultats des concentrations plasmatiques moyennes de curcumine en fonction du temps sont présentés dans le Tableau 1.

**Tableau 1 : Concentration plasmatique de curcumine après administration orale d'une préparation Curcumine S1 chez le cheval**

| Temps (heures) | Nanogrammes/millilitre |
|---|---|
| 0 | 0 |
| 3 | 230 |
| 6 | 260 |
| 9 | 280 |
| 12 | 410 |
| 24 | 350 |
| 36 | 150 |
| 50 | 0 |

### Discussion

Comme on peut le voir, les concentrations plasmatiques de curcumine obtenues obtenus avec la préparation Curcumine S1 sont relativement élevées par rapport à la dose utilisée par voie orale en une seule prise (2.6 grammes pour 500 kg de poids vif, soit 5.6 mg/kg) avec un pic plasmatique à 12 heures et présent de façon quasi constante pendant 12 heures (T12=410 ng/ml et T24= 350 ng/ml).

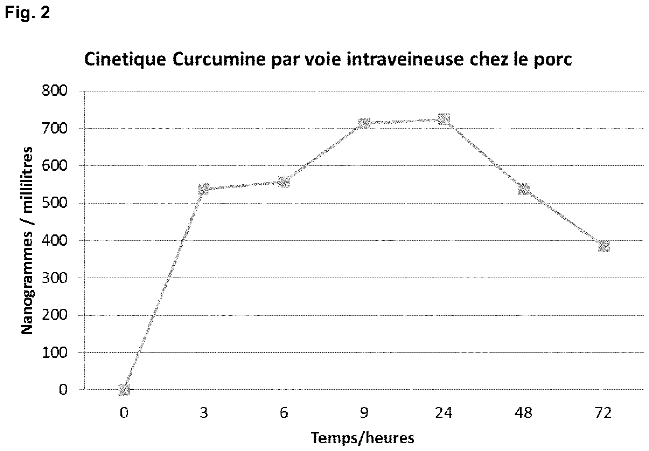

Effectivement, la comparaison avec une étude chez le porc après administration intraveineuse de 0.60 grammes de curcumine pour 23 kg de poids vif avec un pic plasmatique de 24 heures et présent de façon constante pendant 10 heures (Tableau 2, Fig. 2), montre que la Curcumine S1 présente grâce à la présence de spiruline ou d'extraits de spiruline une très bonne biodisponibilité par voie orale avec des effets d'excrétion réduits et en conséquence un pic plasmatique plus rapide (12 heures) qui perdure pendant au moins 12 heures.

**Tableau 2 : Concentration plasmatique de curcumine après administration intraveineuse chez le porc (à titre de comparaison)**

| Temps (heures) | Nanogrammes/millilitre |
|---|---|
| 0 | 0 |
| 3 | 537 |
| 6 | 557 |
| 9 | 713 |
| 24 | 724 |
| 48 | 537 |
| 72 | 385 |

### Conclusions

Les résultats obtenus permettent de conclure que :
1) La Curcumine S1 (préparation selon l'invention) présente une très bonne biodisponibilité de la curcumine avec une demi-vie plasmatique d'au moins 24 heures, en une seule prise orale.
2) La pharmacocinétique de la Curcumine S1 par voie orale est comparativement similaire à la cinétique d'une curcumine utilisée par voie intraveineuse (chez le porc).

## Revendications

1. Utilisation non-thérapeutique d'une quantité efficace de spiruline et/ou d'extrait(s) de spiruline pour améliorer la biodisponibilité de la curcumine prise par voie orale chez l'animal, en particulier le cheval, le porc, le chien et le chat ; ou l'homme et dans laquelle la curcumine est utilisée à titre de complément alimentaire dans le domaine de l'alimentation animale ou humaine.

2. Utilisation non-thérapeutique selon la revendication 1, dans laquelle le rapport pondéral spiruline : curcumine, respectivement le rapport pondéral extrait(s) de spiruline : curcumine, est compris entre 20 : 1 et 1 : 3, de manière davantage préférée entre 10 : 2 et 1 : 2, en particulier entre 8 : 3 et 3 : 4.

3. Utilisation non-thérapeutique selon l'une des revendications 1 ou 2, dans laquelle les quantités de curcumine se situent entre 0,1 et 20 mg/kg, de préférence entre 0,5 et 15 mg/kg, de manière davantage préférée entre 1 et 10 mg/kg de poids corporel de l'animal ou de l'homme.

4. Préparation thérapeutique orale à biodisponibilité améliorée de la curcumine consistant en une combinaison de curcumine avec une quantité efficace de spiruline et/ou un ou des extrait(s) de spiruline.

5. Préparation thérapeutique selon la revendication 4, dans laquelle le rapport pondéral spiruline : curcumine, respectivement le rapport pondéral extrait(s) de spiruline : curcumine, est compris entre 20 : 1 et 1 : 3, de manière davantage préférée entre 10 : 2 et 1 : 2, en particulier entre 8 : 3 et 3 : 4.

6. Préparation thérapeutique selon l'une des revendications 4 ou 5, qui est une préparation orale prête à l'emploi ou une préparation concentrée à mélanger aux aliments usuels ; en tant que préparation liquide ou solide, de préférence en poudre, sous forme de comprimés ou de gélules.

7. Préparation orale à biodisponibilité améliorée de la curcumine comprenant de la curcumine et une quantité efficace de spiruline et/ou d'extrait(s) de spiruline, éventuellement en combinaison avec d'autres ingrédients actifs, excipients ou supports, pour l'utilisation pour le traitement ou la prévention de maladies humaines et animales, choisies parmi les maladies cardiovasculaires, le diabète, les maladies neurologiques et la maladie de Crohn.

8. Préparation pour l'utilisation selon la revendication 7, dans laquelle le rapport pondéral spiruline : curcumine, respectivement le rapport pondéral extrait(s) de spiruline : curcumine, est compris entre 20 : 1 et 1 : 3, de manière davantage préférée entre 10 : 2 et 1 : 2, en particulier entre 8 : 3 et 3 : 4.

9. Préparation pour l'utilisation selon l'une des revendications 7 ou 8, dans laquelle les quantités de curcumine se situent entre 0,1 et 20 mg/kg, de préférence entre 0,5 et 15 mg/kg, de manière davantage préférée entre 1 et 10 mg/kg de poids corporel de l'animal ou de l'homme.

10. Préparation non-thérapeutique orale à biodisponibilité améliorée de la curcumine, notamment un complément alimentaire, consistant en une combinaison de curcumine avec une quantité efficace de spiruline et/ou un ou des extrait(s) de spiruline.

11. Préparation non-thérapeutique selon la revendication 10, dans laquelle le rapport pondéral spiruline : curcumine, respectivement le rapport pondéral extrait(s) de spiruline : curcumine, est compris entre 20 : 1 et 1 : 3, de manière davantage préférée entre 10 : 2 et 1 : 2, en particulier entre 8 : 3 et 3 : 4.

12. Préparation non-thérapeutique selon l'une des revendications 10 ou 11, qui est une préparation orale prête à l'emploi ou une préparation concentrée à mélanger aux aliments usuels ; en tant que préparation liquide ou solide, de préférence en poudre, sous forme de comprimés ou de gélules.

13. Procédé de fabrication d'une préparation selon l'une des revendications 4 à 6 ou 10 à 12, comprenant l'étape de mélange d'une quantité efficace de spiruline et/ou d'un ou de plusieurs extraits de spiruline avec de la curcumine.

## Patentansprüche

1. Nichttherapeutische Verwendung einer wirksamen Menge von Spirulina und/oder eines oder mehrerer Spirulina-Extrakte zur Verbesserung der Bioverfügbarkeit von Kurkumin, das oral von einem Tier, insbesondere einem Pferd, einem Schwein, einem Hund und einer Katze; oder einem Menschen eingenommen wird, und wobei das Kurkumin als Nahrungsergänzungsmittel im Bereich der Tier- oder menschlichen Ernährung verwendet wird.

2. Nichttherapeutische Verwendung nach Anspruch 1, wobei das Gewichtsverhältnis von Spirulina:Kurkumin bzw. das Gewichtsverhältnis von Spirulina-Extrakt(en):Kurkumin zwischen 20:1 und 1:3, vorteilhaft vorzugsweise zwischen 10:2 und 1:2, insbesondere zwischen 8:3 und 3:4 liegt.

3. Nichttherapeutische Verwendung nach einem der Ansprüche 1 oder 2, wobei die Kurkuminmengen zwischen 0,1 und 20 mg/kg, vorzugsweise zwischen 0,5 und 15 mg/kg, vorteilhaft vorzugsweise zwischen 1 und 10 mg/kg des Körpergewichts des Tiers oder des Menschen liegen.

4. Orales therapeutisches Präparat mit verbesserter Bioverfügbarkeit von Kurkumin, bestehend in einer Kombination von Kurkumin mit einer wirksamen Menge von Spirulina und/oder eines oder mehrerer Spirulina-Extrakte.

5. Therapeutisches Präparat nach Anspruch 4, wobei das Gewichtsverhältnis von Spirulina:Kurkumin bzw. das Gewichtsverhältnis von Spirulina-Extrakt(en):Kurkumin zwischen 20:1 und 1:3, vorteilhaft vorzugsweise zwischen 10:2 und 1:2, insbesondere zwischen 8:3 und 3:4 liegt.

6. Therapeutisches Präparat nach einem der Ansprüche 4 oder 5, das ein gebrauchsfertiges orales Präparat oder ein konzentriertes Präparat zum Mischen mit üblichen Nahrungsmitteln ist; als flüssiges oder festes Präparat, vorzugsweise als Pulver, in der Form von Tabletten oder Kapseln.

7. Orales Präparat mit verbesserter Bioverfügbarkeit von Kurkumin, umfassend Kurkumin und eine wirksame Menge von Spirulina und/oder eines oder mehrerer Spirulina-Extrakte, gegebenenfalls in Kombination mit anderen Wirkbestandteilen, Hilfsstoffen oder Trägerstoffen, zur Verwendung bei der Behandlung oder Verhinderungen von Krankheiten des Menschen oder des Tiers, die aus Herz-Kreislauf-Krankheiten, Diabetes, neurologischen Krankheiten und Morbus Crohn ausgewählt sind.

8. Präparat zur Verwendung nach Anspruch 7, wobei das Gewichtsverhältnis von Spirulina:Kurkumin bzw. das Gewichtsverhältnis von Spirulina-Extrakt(en):Kurkumin zwischen 20:1 und 1:3, vorteilhaft vorzugsweise zwischen 10:2 und 1:2, insbesondere zwischen 8:3 und 3:4 liegt.

9. Präparat zur Verwendung nach einem der Ansprüche 7 oder 8, wobei die Kurkuminmengen zwischen 0,1 und 20 mg/kg, vorzugsweise zwischen 0,5 und 15 mg/kg, vorteilhaft vorzugsweise zwischen 1 und 10 mg/kg des Körpergewichts des Tiers oder des Menschen liegen.

10. Orales nichttherapeutisches Präparat mit verbesserter Bioverfügbarkeit von Kurkumin, insbesondere ein Nahrungsergänzungsmittel, bestehend in einer Kombination von Kurkumin mit einer wirksamen Menge von Spirulina und/oder eines oder mehrerer Spirulina-Extrakte.

11. Nichttherapeutisches Präparat nach Anspruch 10, wobei das Gewichtsverhältnis von Spirulina:Kurkumin bzw. das Gewichtsverhältnis von Spirulina-Extrakt(en):Kurkumin zwischen 20:1 und 1:3, vorteilhaft vorzugsweise zwischen 10:2 und 1:2, insbesondere zwischen 8:3 und 3:4 liegt.

12. Nichttherapeutisches Präparat nach einem der Ansprüche 10 oder 11, das ein gebrauchsfertiges orales Präparat oder ein konzentriertes Präparat zum Mischen mit üblichen Nahrungsmitteln ist; als flüssiges oder festes Präparat, vorzugsweise als Pulver, in der Form von Tabletten oder Kapseln.

13. Verfahren zur Herstellung eines Präparats nach einem der Ansprüche 4 bis 6 oder 10 bis 12, umfassend den Schritt des Mischens einer wirksamen Menge von Spirulina und/oder eines oder mehrerer Spirulina-Extrakte mit dem Kurkumin.

## Claims

1. Non-therapeutic use of an effective quantity of spirulina and/or of spirulina extract(s) for improving the bioavailability of orally administered curcumin in animals, in particular horses, pigs, dogs and cats or humans and wherein the curcumin is used as a food supplement in the field of animal or human nutrition.

2. Non-therapeutic use according to claim 1, wherein the weight ratio of spirulina:curcumin, or the weight ratio of spirulina extract(s):curcumin, is comprised between 20:1 and 1:3, more preferably between 10:2 and 1:2 and in particular between 8:3 and 3:4.

3. Non-therapeutic use according to one of claims 1 or 2, wherein the quantities of spirulina range between 0.1 and 20 mg/kg, preferably between 0.5 and 15 mg/kg and more preferably between 1 and 10 mg/kg body weight of the animal or human.

4. Therapeutic oral preparation with improved bioavailability of curcumin consisting of a combination of curcumin and an effective quantity of spirulina and/or spirulina extract(s).

5. Therapeutic preparation according to claim 4, wherein the weight ratio of spirulina:curcumin, or the weight ratio of spirulina extract(s):curcumin, is comprised between 20:1 and 1:3, more preferably between 10:2 and 1:2 and in particular between 8:3 and 3:4.

6. Therapeutic preparation according to one of claims 4 or 5, which is a ready-to-use oral preparation or concentrated preparation to be mixed with ordinary foods, as a liquid or solid preparation, preferably as a powder, in the form of tablets or capsules.

7. Oral preparation with improved bioavailability of curcumin comprising curcumin and an effective quantity of spirulina and/or spirulina extract(s), possibly in combination with other active ingredients, excipients or supports. for use in the treatment or the prevention of human and animal diseases, selected from cardiovascular diseases, diabetes, neurological diseases and Crohn's disease.

8. Preparation for use according to claim 7, wherein the weight ratio of spirulina:curcumin, or the weight ratio of spirulina extract(s):curcumin, is comprised between 20:1 and 1:3, more preferably between 10:2 and 1:2 and in particular between 8:3 and 3:4.

9. Preparation for use according to one of claims 7 or 8, wherein the quantities of curcumin range between 0.1 and 20 mg/kg, preferably between 0.5 and 15 mg/kg and more preferably between 1 and 10 mg/kg body weight of the animal or human.

10. Non-therapeutic oral preparation with improved bioavailability of curcumin, particularly a food supplement, consisting of a combination of curcumin and an effective quantity of spirulina and/or spirulina extract(s).

11. Non-therapeutic preparation according to claim 10, wherein the weight ratio of spirulina:curcumin, or the weight ratio of spirulina extract(s):curcumin, is comprised between 20:1 and 1:3, more preferably between 10:2 and 1:2 and in particular between 8:3 and 3:4.

12. Non-therapeutic preparation according to one of claims 10 or 11, which is a ready-to-use oral preparation or a concentrated preparation to be mixed with ordinary foods, as a liquid or solid preparation, preferably as a powder, in the form of tablets or capsules.

13. Manufacturing process for a preparation according to one of claims 4 to 6 or 10 to 12, comprising the step of mixing an effective quantity of spirulina and/or one or more spirulina extracts with curcumin.
